# DEMANDE DE BREVET EUROPEEN

(11) **EP 3 771 896 A1**
(43) Date de publication de la demande: **03.02.2021**
(21) Numéro de dépôt: 20182073.5
(22) Date de dépôt: 24.06.2020
(51) Int. Cl.: G01N 1/31, G01N 35/00, G01N 1/36

(54) **INSTALLATION ET PROCÉDÉ POUR LE CONTRÔLE DE L'ORIGINE ET/OU DE LA QUALITÉ DE COUPES D'ÉCHANTILLONS BIOLOGIQUES**

(30) Priorité: 29.07.2019 FR 1908590
(71) Demandeur: Dreampath Diagnostics, 67100 Strasbourg (FR)
(72) Inventeur: JORDAN NAVAS, Pablo, 28005 MADRID (ES); WILHELM, Valérie, 67114 ESCHAU (FR)
(74) Mandataire: Hugues, Catherine

(57) **Abrégé**

La présente invention concerne une installation (1) pour le contrôle de l'origine et/ou de la qualité de coupes (2) d'échantillons biologiques (7) stockées sur des lames porte-objet (3), comportant :
- au moins un dispositif (9, 12) de lecture de données d'identification encodées,
- au moins un dispositif d'acquisition de données biométriques propres à un échantillon biologique (7),
- des moyens de transmission (13) des données vers des moyens de traitement informatiques (14).

L'invention concerne également l'utilisation d'une telle installation pour le contrôle de l'origine et/ou de la qualité de lames d'échantillons biologiques ou pour le contrôle de la qualité de la coloration de lames d'échantillons biologique, ainsi que des procédés de mise en œuvre de l'installation dans le cadre de telles utilisations.

## Description

La présente invention a pour objet une installation pour le contrôle de l'origine et/ou de la qualité de coupes d'échantillons biologiques, stockées sur des lames porte-objets munies de données d'identification encodées et prélevées sur des blocs de paraffine également munis de données d'identification encodées.

Dans le cadre de la prise en charge médicale d'un patient ou celui de travaux de recherche dans le domaine humain, animal ou végétal, des prélèvements tissulaires ou cellulaires sont couramment effectués en vue d'une analyse histologique et/ou moléculaire, destinée ou non à l'établissement d'un diagnostic.

Afin de permettre une parfaite visualisation au microscope des structures cellulaires ou tissulaires analysées, de tels prélèvements font l'objet d'une préparation préalable comportant différentes étapes.

De manière résumée, au cours de ces dernières, les échantillons frais prélevés sont placés dans des cassettes ajourées, subissent une phase de déshydratation, puis une inclusion en paraffine conduisant à des blocs d'échantillons biologiques. Des coupes fines et régulières, de 4 à 10 µm d'épaisseur, prélevées à partir de ces derniers, sont ensuite placées sur des lames porte-objet, subissent encore différents traitements à l'aide de produits chimiques et de colorants afin de révéler les structures à observer, et sont enfin recouvertes d'une colle puis d'une lamelle permettant leur lecture au microscope et assurant leur protection.

Il est notoire qu'en raison du vieillissement de la population et de la croissance du taux d'incidence de certaines maladies telles que le cancer, les laboratoires de pathologie sont aujourd'hui amenés à gérer un très grand nombre d'échantillons biologiques, par exemple sous forme de biopsies, ou de pièces opératoires.

Les biopsies sont généralement petites, voire très petites et conduisent généralement, après traitement et déshydratation, à un unique ou petit nombre de blocs de paraffine. Au contraire, les pièces opératoires peuvent être volumineuses, notamment lorsqu'elles consistent en un organe entier, de sorte qu'il peut être nécessaire de les fractionner, ce qui conduit alors à une pluralité de blocs de paraffine intégrant chacun une des fractions obtenues.

Toutes les étapes de préparation des échantillons biologiques en vue de leur analyse supposent de nombreuses manipulations, au cours desquelles des mesures doivent être mises en œuvre pour garantir non seulement l'exactitude des analyses effectuées et en particulier des diagnostics établis, mais également une correspondance entre leur origine réelle et celle qui leur est attribuée au cours de leur parcours.

Ainsi, dès son prélèvement, chaque échantillon biologique se voit attribuer un numéro de référence unique, fourni par les systèmes informatiques des laboratoires, le liant au patient dont il provient, et qui va le suivre tout au long de son parcours, qu'il soit stocké dans un pot, une cassette, un bloc de paraffine ou sur une lame porte-objet. Par ailleurs, chaque prélèvement (biopsie ou fragment de pièce opératoire) présente une forme unique, parfois décelable à l'œil nu, mais dans d'autres cas uniquement par analyse microscopique.

A l'heure actuelle, l'opération consistant à vérifier si le numéro de référence d'une lame porte-objet, supposée porter un échantillon biologique issu d'une coupe d'un bloc donné de paraffine, concorde avec le numéro de référence de ce dernier et émane donc du même patient, s'effectue principalement par contrôle visuel. En guise de double sécurité, et lorsque l'échantillon l'autorise, un contrôle visuel est parfois effectué pour déterminer si la forme de l'échantillon placé sur une lame porte-objet correspond à celle de l'échantillon intégré dans le bloc de paraffine.

Toutefois, une telle méthode atteint actuellement ses limites, en raison des volumes d'échantillons toujours plus importants à gérer par les laboratoires de pathologie anatomique, et peut conduire à des erreurs, en particulier lors de l'octroi des numéros de référence aux lames porte-objet au moment de leur fabrication.

Dans certains cas, les pathologistes sont heureusement en mesure de détecter de telles erreurs lors de leur intervention, notamment si le profil du patient à qui une lame a été attribuée ne correspond pas à celui de l'échantillon stocké sur cette même lame, ou si la forme de l'échantillon stocké sur la lame diffère de celle de l'échantillon inclus dans le bloc. Néanmoins, le laboratoire doit alors rechercher le bloc de paraffine d'origine, le recouper, le colorer, etc., ce qui nécessite un temps de travail et un coût supplémentaires, et surtout un retard dans le diagnostic du patient. Et, lorsqu'au contraire l'erreur est impossible à détecter, par exemple parce que le profil du patient correspondant, identifié via le numéro de référence porté par la lame porte-objet, est susceptible d'être compatible avec l'échantillon biologique analysé, le pathologiste fournira un diagnostic qui sera malheureusement délivré à un mauvais patient. Cela peut évidemment être fatal, à la fois pour ce dernier s'il bénéficie alors de soins et/ou d'un traitement inappropriés ayant des effets secondaires, et pour le patient réellement à l'origine de l'échantillon analysé qui ne bénéficiera alors ni du diagnostic ni de quelconques soins.

D'autre part, il a également été observé que des erreurs de diagnostic peuvent résulter d'un défaut de qualité des lames porte-objet analysées.

En effet, la fabrication de ces dernières est particulièrement délicate, nécessite une grande dextérité de la part des techniciens concernés, et peut être affectée par de nombreux paramètres tels que la mauvaise qualité de la lame utilisée, la fatigue du technicien, la température ambiante, etc. Il arrive par ailleurs que la coupe ne soit pas effectuée suffisamment profondément dans le bloc de paraffine, de sorte qu'une partie de l'échantillon, susceptible de présenter un intérêt majeur pour l'établissement du diagnostic, fait finalement défaut sur la lame porte-objet obtenue.

Une mauvaise qualité d'une lame porte-objet peut en outre résulter d'une coloration approximative de l'échantillon biologique qui y est stocké, au moyen de solutions chimiques aux propriétés dégradées. En raison du très grand nombre de lames porte-objet colorées quotidiennement, la qualité des réactifs et des consommables présents dans les bains diminue en effet au fur et à mesure de leur utilisation. Ainsi, si les colorants, les alcools et les autres produits chimiques ne sont pas changés et rafraîchis régulièrement, la qualité de la coloration en termes de netteté, contraste, intensité de la couleur, diminue après chaque lot de lames traité. Or, cette baisse de qualité de coloration d'une lame porte-objet est très difficile à détecter à l'œil nu car elle se produit très progressivement. Un autre défaut de lisibilité peut être dû à l'absence d'étalement correct de la colle sur la coupe rendant la lecture au microscope impossible. Cette faible qualité des lames due à une préparation négligente peut rendre les diagnostics effectués par le pathologiste plus difficiles, voire même entraîner des diagnostics erronés, certains détails ou particularités des tissus n'ayant pas été révélés et demeurant par conséquent indécelables.

L'objet de la présente invention est de proposer une solution permettant de pallier l'ensemble des problèmes décrits ci-dessus, et par conséquent de garantir la fiabilité des diagnostics établis par les laboratoires de pathologie, aussi bien en termes de précision qu'en termes de destinataire, notamment au travers d'un double contrôle quant à l'origine des échantillons biologiques stockées sur des lames porte-objet, effectué immédiatement après la fabrication de ces dernières. De plus, l'objet de l'invention est également de permettre un contrôle de la qualité des lames porte-objet dès leur fabrication, permettant de n'acheminer vers les pathologistes en charge des opérations d'analyse et d'établissement de diagnostics, que des lames porte-objet présentant la qualité de préparation requise.

A cet effet, la présente invention concerne une installation pour le contrôle de l'origine et/ou de la qualité de coupes d'échantillons biologiques, prélevées sur des blocs de paraffine munis de données d'identification encodées et stockées sur des lames porte-objets également munies de données d'identification encodées, ladite installation comportant :
- au moins un dispositif de lecture de données d'identification encodées, caractérisée en ce qu'elle comporte en outre :
   - au moins un dispositif d'acquisition de données biométriques propres à un échantillon biologique inclus dans un bloc de paraffine ou propres à un échantillon biologique stocké sur une lame porte-objet sous forme d'une coupe prélevée à partir d'un bloc de paraffine,
   - des moyens de transmission des données d'identification relevées et des données biométriques acquises vers des moyens de traitement informatiques intégrant une application logicielle exécutée pour
      * enregistrer de manière associée dans des moyens mémoire les données d'identification relevées sur un bloc de paraffine et les données biométriques propres à l'échantillon inclus dans ledit bloc de paraffine et préalablement acquises,
      * enregistrer les données d'identification relevées sur une lame porte-objet et les comparer avec celles enregistrées pour ledit bloc de paraffine, et, le cas échéant
      * enregistrer les données biométriques propres à l'échantillon biologique stocké sous forme de coupe sur ladite lame porte-objet et les comparer avec les données biométriques enregistrées de manière associée avec lesdites données d'identification dudit bloc de paraffine, et
      * activer des moyens de genèse d'un signal représentatif du résultat de la ou des comparaisons.

Conformément à une variante de réalisation préférentielle, l'installation selon l'invention comporte un appareil présentant un logement conformé pour recevoir au moins un bloc de paraffine ou au moins une lame porte-objet et dans lequel sont installés de manière appropriée au moins un dispositif de lecture de données encodées destiné à relever les données d'identification d'un bloc de paraffine, au moins un second dispositif de lecture de données encodées destiné à relever les données d'identification d'une lame porte-objet, ainsi qu'au moins un dispositif d'acquisition de données biométriques destiné à acquérir les données biométriques propres à un échantillon biologique inclus dans un bloc de paraffine et/ou les données biométriques propres à un échantillon biologique stocké sous forme de coupe sur une lame porte-objet.

Une autre variante de réalisation peut encore être envisagée dans laquelle ledit appareil comporte un dispositif d'acquisition de données biométriques destiné à acquérir les données biométriques propres à un échantillon biologique inclus dans un bloc de paraffine et un dispositif d'acquisition de données biométriques destiné à acquérir les données biométriques propres à un échantillon biologique stocké sous forme de coupe sur une lame porte-objet.

L'installation comporte en plus d'un tel appareil, un ordinateur pourvu des moyens de traitement informatique et d'au moins une interface utilisateur, ledit ordinateur étant relié audit appareil par une liaison filaire ou non filaire.

Selon une autre option imaginée, l'appareil intègre lui-même les moyens de traitement informatiques et au moins une interface utilisateur.

De préférence, le/les dispositif(s) d'acquisition de données biométriques est/sont défini(s) par un dispositif d'acquisition de données relatives à une forme bidimensionnelle ou tridimensionnelle propre à un échantillon biologique inclus dans un bloc de paraffine ou propre à un échantillon biologique stocké, sous forme d'une coupe prélevée à partir d'un bloc de paraffine, sur une lame porte-objet.

D'autre part, l'installation selon l'invention se caractérise encore par le fait qu'elle comporte au moins un lecteur de codes-barres permettant de lire les données d'identification encodées apposées sur un bloc de paraffine ou sur une lame porte-objet.

Il a par ailleurs été prévu que l'interface utilisateur consiste de préférence en un écran.

L'invention a également pour objet un procédé de contrôle de l'origine et/ou de la qualité de coupes d'échantillons biologiques, prélevées sur des blocs de paraffine munis de données d'identification encodées, lesdites coupes étant stockées sur des lames porte-objet également munies de données d'identification encodées, au moyen d'une installation telle que décrite ci-dessus, caractérisé par la réalisation, dans l'ordre, des étapes suivantes:
- relever les données d'identification d'au moins un bloc de paraffine au moyen d'un dispositif de lecture de données encodées,
- acquérir les données biométriques propres à l'échantillon biologique inclus dans ce même bloc de paraffine au moyen d'un dispositif d'acquisition de données biométriques,
- enregistrer de manière associée les données d'identification du bloc de paraffine et les données biométriques propres à l'échantillon biologique inclus dans ce même bloc de paraffine dans des moyens-mémoire,
- relever les données d'identification d'au moins une lame porte-objet au moyen d'un dispositif de lecture de données encodées,
- acquérir les données biométriques propres à l'échantillon biologique stocké sous forme de coupe dans ladite lame porte-objet au moyen d'un dispositif d'acquisition de données biométriques,
- comparer les données d'identification de ladite lame porte-objet et les données d'identification dudit bloc de paraffine puis, comparer les données biométriques propres à l'échantillon biologique stocké sous forme de coupe dans ladite lame porte-objet et les données biométriques propres à l'échantillon biologique inclus dans ledit bloc de paraffine
- réceptionner un signal représentatif du résultat des comparaisons effectuées, , et, selon le cas
- Confirmer l'origine et/ou la qualité de l'échantillon stocké sur la lame porte-objet, ou
- Infirmer l'origine et/ou la qualité de l'échantillon stocké sur la lame porte-objet.

D'autre part, l'invention a également pour objet un procédé de contrôle de la qualité de la coloration d'une lame-objet au moyen d'une installation telle que précédemment décrite, caractérisé par la réalisation des étapes suivantes :
- relever les données d'identification d'au moins une lame porte-objet, dite « lame porte-objet de référence », au moyen d'un dispositif de lecture de données encodées,
- acquérir les données biométriques propres à l'échantillon biologique stocké sous forme de coupe convenablement colorée dans la lame porte-objet « de référence », au moyen d'un dispositif d'acquisition de données biométriques,
- enregistrer de manière associée les données d'identification de la lame porte-objet « de référence » et les données biométriques propres à l'échantillon biologique stocké sous forme de coupe convenablement colorée dans la lame porte-objet « de référence », dans des moyens-mémoire,
- relever les données d'identification d'une lame porte-objet au moyen d'un dispositif de lecture de données encodées,
- acquérir les données biométriques propres à l'échantillon biologique stocké sous forme de coupe colorée dans ladite lame porte-objet au moyen d'un dispositif d'acquisition de données biométriques,
- réceptionner un signal représentatif du résultat d'une comparaison entre les données biométriques propres à l'échantillon biologique stocké sous forme de coupe colorée dans ladite lame porte-objet et les données biométriques propres à l'échantillon biologique stocké sous forme de coupe convenablement colorée dans ladite lame porte-objet « de référence », effectuée(s) par des moyens de traitement informatique, et, selon le cas
- Confirmer la qualité de la coloration de l'échantillon biologique stocké sous forme de coupe colorée dans la lame porte-objet et conserver la lame porte-objet (3) telle quelle, ou
- Infirmer la qualité de la coloration de l'échantillon biologique stocké sous forme de coupe colorée dans la lame porte-objet et soumettre la lame porte-objet à une nouvelle étape de coloration, ou détruire la lame porte-objet.

L'invention s'étend également à l'utilisation de l'installation telle que décrite ci-dessus pour contrôler l'origine et/ou la qualité de coupes d'échantillons biologiques, prélevées sur des blocs de paraffine et stockées sur des lames porte-objet ou pour contrôler la coloration de coupes d'échantillons biologiques stockées sur des lames porte-objet.

La présente invention sera mieux comprise à la lecture de la description faite en référence aux figures annexées, fournies à titre d'exemples non limitatifs, et dans lesquelles :

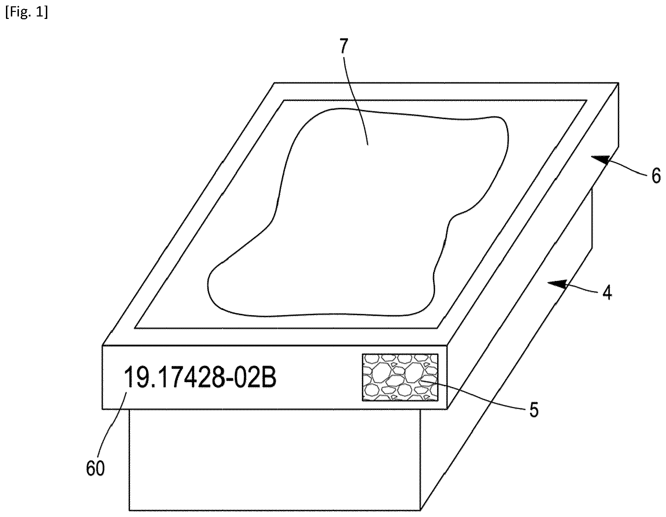

- La figure 1 illustre une vue en perspective d'un bloc de paraffine intégrant un échantillon biologique,

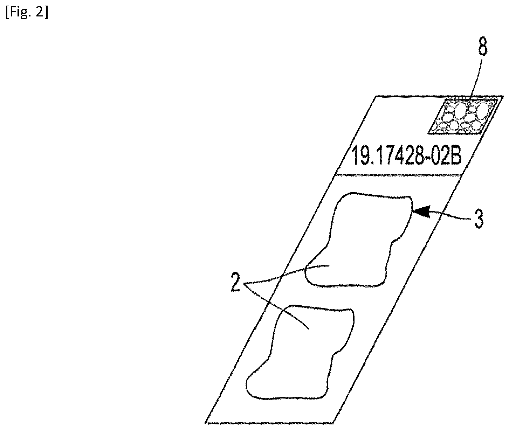

- La figure 2 est une vue d'une lame porte-objet sur laquelle est stockée une coupe prélevée sur le bloc de paraffine de la figure 1, et
- La figure 3 est une vue schématique d'une variante de réalisation de l'installation selon l'invention lors d'une étape du procédé de contrôle de l'origine et/ou de la qualité d'une coupe d'échantillon biologique prélevée sur le bloc de paraffine de la figure 1 et stockée sur la lame porte-objet de la figure 2.

En référence aux figures, la présente invention a pour objet une installation 1 pour le contrôle de l'origine et/ou de la qualité de coupes 2 d'échantillons biologiques stockées sur des lames porte-objets 3, et prélevées sur des blocs de paraffine 4.

De manière classique, des données d'identification, représentatives du patient concerné, sont attribuées dès leur prélèvement à l'ensemble des échantillons biologiques destinés à la réalisation d'analyses et/ou à l'établissement de diagnostics, et vont les accompagner tout au long de leur parcours à travers les différents services susceptibles d'intervenir, quel que soit le type de support sur lequel ils sont stockés.

Ainsi, chaque bloc de paraffine 4 est muni de telles données d'identification, généralement apposées sur une face 60 d'une cassette 6 le contenant, lesdites données se présentant par exemple sous forme de code matriciel 5, et permettant de relier chaque bloc de paraffine 4 au patient dont provient l'échantillon biologique 7 qu'il intègre. Il va de soi que l'ensemble des blocs de paraffine 4, intégrant chacun une fraction d'un organe provenant d'un seul et même patient, sont supposés porter un code matriciel 5 unique.

De même, chaque lame porte-objet 3 sur laquelle est stockée une coupe 2 prélevée sur un bloc de paraffine 4 est munie de données d'identification unique, par exemple sous forme de code matriciel 8. Ainsi, afin de garantir l'émission d'un diagnostic vers le patient à l'origine du prélèvement, le code matriciel 8 d'une lame porte-objet 3 fabriquée à partir d'un bloc de paraffine 4 doit nécessairement correspondre au code matriciel 5 apposé sur ce dernier.

La mise en œuvre de l'installation 1 selon l'invention lors de la fabrication de lames porte-objet 3 permet d'éviter ou de déceler toute apposition d'un code matriciel 8 erroné sur ces dernières.

En d'autres termes, la mise en œuvre de l'installation 1 immédiatement après la fabrication de lames porte-objet 3 permet de ne mettre à disposition des pathologistes en charge des analyses que des lames porte-objet 3 pour lesquelles l'identité du patient a été préalablement contrôlée.

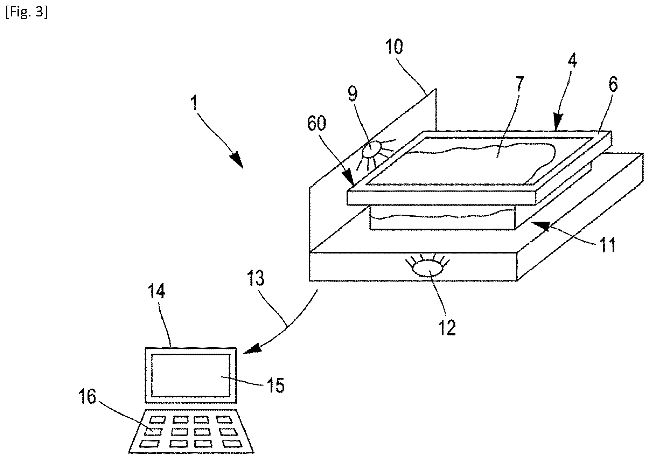

A cet effet, dans la variante de réalisation illustrée à la figure 3, l'installation comporte un appareil 10 présentant un logement 11 conformé pour recevoir au moins un bloc de paraffine 4 ou au moins une lame porte-objet 3 et dans lequel sont installés de manière appropriée un premier lecteur 9 de code-barres destiné à relever le code matriciel 5 apposé sur une face 60 d'une cassette 6 contenant un bloc de paraffine 4, et un second lecteur 12 de code-barres destiné à relever le code matriciel 8 d'une lame porte-objet 3. Un tel appareil peut être défini par un dispositif d'acquisition et d'analyse d'images à la résolution adéquate.

Par ailleurs, l'appareil 10 intègre encore au moins un dispositif (non illustré) d'acquisition de données relatives à une forme bidimensionnelle ou tridimensionnelle propre à un échantillon biologique 7 inclus dans un bloc de paraffine 4 ou propre à un échantillon biologique 7 stocké sous forme d'une coupe 2 prélevée à partir d'un bloc de paraffine 4 sur une lame porte-objet 3.

En somme, l'appareil 1 est équipé de moyens lui permettant de lire un code-barre apposé sur un bloc de paraffine 4, de moyens lui permettant de lire un code-barre apposé sur une lame porte-objet 3, de moyens lui permettant de déterminer une forme caractéristique d'un échantillon biologique 7 stocké dans un bloc de paraffine 4, et de moyens lui permettant de déterminer une forme caractéristique d'un échantillon biologique 7 stocké sur une lame porte-objet 3, sous forme de coupe 2 prélevée à partir de ce même bloc 4.

L'installation 1 comprend également un ordinateur 14, pourvu d'un écran 15 et d'un clavier 16, et relié à l'appareil 1 par des moyens de transmission de données 13, filaires ou non filaires.

Conformément à l'invention, l'ordinateur 14 intègre des moyens de traitement informatiques intégrant une application logicielle exécutée pour, dans la variante décrite :
* enregistrer de manière associée dans des moyens mémoire les données d'identification relevées à partir du code matriciel 5 sur un bloc de paraffine 4 et les données relatives à la forme caractéristique de l'échantillon biologique 7 inclus dans ce même bloc de paraffine 4,
* enregistrer les données d'identification relevées à partir du code matriciel 8 sur une lame porte-objet 3 et les comparer avec celles enregistrées pour ledit bloc de paraffine 4, et, le cas échéant
* enregistrer les données relatives à la forme caractéristique de l'échantillon biologique 7 stocké sous forme de coupe 2 sur ladite lame porte-objet 3 et les comparer avec les données relatives à la forme caractéristique de l'échantillon biologique 7 inclus dans le bloc de paraffine 4 enregistrées de manière associée avec les données d'identification dudit bloc de paraffine 4, et
* activer des moyens de genèse d'un signal représentatif du résultat de la ou des comparaisons, par l'exemple sous la forme d'un signal visuel apparaissant sur l'écran 15 de l'ordinateur 14.

En pratique, l'installation selon l'invention peut être utilisée dans le cadre de l'application décrite ci-dessus pour contrôler de manière automatisée l'origine d'un échantillon biologique 7 stocké sous forme de coupe 2 sur une lame porte-objet 3.

Ce contrôle s'effectue avantageusement à deux niveaux, à savoir au travers d'une comparaison entre les données d'identification apposées sur la lame porte-objet 3 et celles apposées sur le bloc de paraffine 4 ayant servi à sa fabrication, et d'une seconde comparaison effectuée cette fois entre la forme de l'échantillon 7 supporté par la lame 3 et celle de l'échantillon 7 inclus dans le bloc de paraffine 4. La comparaison des formes permet de surcroît avantageusement de déceler d'éventuels défauts de coupe (tels que des plis de tissu ou des traces de lames, etc.) et notamment des coupes effectuées à des profondeurs insuffisantes dans les blocs de paraffine et susceptibles de donner lieu à des diagnostics erronés.

Dans ce contexte, la mise en œuvre de l'installation 1 consiste à placer tout d'abord un bloc de paraffine 4 dans le logement 11 de l'appareil 10 de l'installation 1, puis à lire le code matriciel 5 d'un bloc de paraffine 4 au moyen du lecteur de code-barres 9 et ensuite déterminer une forme propre à l'échantillon biologique 7 inclus dans ce même bloc de paraffine 4 au moyen d'un dispositif d'acquisition et d'analyse d'image à la résolution adéquate (non illustré).

Les données d'identification du bloc de paraffine 4 relevées et les données relatives à la forme propre à l'échantillon biologique 7 inclus dans ce même bloc de paraffine 4 sont alors automatiquement enregistrées de manière associée dans les moyens mémoire de l'ordinateur 14.

Puis, le bloc de paraffine 4 est extrait du logement 11 de l'appareil 10, et remplacé par une lame porte-objet 3 fabriquée à partir dudit bloc de paraffine 4, et dont l'origine et la qualité doivent être contrôlés. A cet effet, il convient de relever à présent les données d'identification de la lame porte-objet 3, au moyen du lecteur de code-barre 12, puis de déterminer une forme propre à l'échantillon biologique 7 stocké sous forme de coupe 2 dans ladite lame porte-objet 3 au moyen dudit dispositif d'acquisition et d'analyse d'image.

Les moyens de traitement informatique de l'ordinateur 14 génèrent alors un signal représentatif du résultat d'une comparaison entre le code matriciel 8 de ladite lame porte-objet 3 et le code matriciel 5 dudit bloc de paraffine 4 et, le cas échéant d'une comparaison entre la forme propre à l'échantillon biologique 7 stocké sous forme de coupe 2 dans ladite lame porte-objet 3 et la forme propre à l'échantillon biologique 7 inclus dans ledit bloc de paraffine 4, effectuée(s) par des moyens de traitement informatique. Ce signal s'affiche sur l'écran 15, et, selon le cas correspondra à une confirmation de l'origine et/ou de la qualité de la lame porte-objet 3, ou à une infirmation de l'origine et/ou la qualité de la lame porte-objet 3.

En cas de détection d'une concordance insuffisante entre la forme de l'échantillon 7 présent sur la lame porte-objet 3 et celle de l'échantillon 7 inclus dans le bloc de paraffine 5, ou d'une coupe 2 réalisée à une profondeur insuffisante, l'opérateur ayant procédé à la fabrication de de la lame porte-objet 3 sera immédiatement averti, dès la fin de cette fabrication. Ceci lui permettra de déceler une éventuelle erreur d'attribution d'une lame 3 à un mauvais patient ou de constater un défaut de fabrication nécessitant des recoupes complémentaires.

Une comparaison des formes peut également permettre de déceler d'éventuels plis de tissu ou des traces de lame de microtome présents dans un échantillon biologique stocké dans une coupe 2 sur une lame porte-objet 3, et également susceptibles de mener à des erreurs de diagnostic. Les lames porte-objet 3 portant de telles coupes 2 pourront ainsi également être supprimées dès le contrôle effectué via l'installation 1, sans être transmises au pathologiste en charge de leur analyse.

Il peut également être prévu de détruire toute lame porte-objet 3 dès qu'une erreur, quelle qu'elle soit, est décelée, afin d'éviter toute future erreur de diagnostic ou d'attribution de ce dernier à un mauvais destinataire.

Par ailleurs, l'installation 1 peut également être mise en œuvre uniquement pour vérifier la qualité de la coloration d'une coupe 2 stockée sur une lame porte-objet 3. Dans ce cas, il est prévu d'utiliser une lame porte-objet dite « de référence » pour laquelle il est établi que l'ensemble des éléments susceptibles d'être observés sur l'échantillon 7 ont bien été révélés suite aux traitements chimiques réalisés.

Dans le cadre de cette application, il est prévu en pratique de disposer d'abord dans le logement 11 de l'appareil 10 une lame porte-objet « de référence » pour acquérir la forme propre à un échantillon biologique 7 stocké sous forme de coupe 2 convenablement colorée dans cette même lame porte-objet « de référence », au moyen du dispositif d'acquisition et d'analyse d'images, après avoir lu le code matriciel de ladite lame de référence. L'image obtenue est alors enregistrée dans les moyens-mémoire de l'ordinateur 14, associée aux données d'identification de la lame porte-objet de référence. Puis la lame porte-objet « de référence » est extraite du logement 11 et remplacée par la lame porte-objet 3 dont on veut vérifier la qualité de la coloration. Le lecteur de code-barres 12 est à nouveau activé, puis le dispositif d'acquisition et d'analyse d'images est sollicité, pour déterminer cette fois la forme propre à l'échantillon biologique 7 stocké sous forme de coupe 2 colorée dans la lame porte-objet 3.

Un signal s'affiche alors sur l'écran 15 de l'ordinateur 14. Il est représentatif du résultat d'une comparaison entre la forme propre à l'échantillon biologique 7 stocké sous forme de coupe 2 colorée dans la lame porte-objet 3 et la forme propre à l'échantillon biologique 7 stocké sous forme de coupe 2 convenablement colorée dans la lame porte-objet « de référence », effectuée par les moyens de traitement informatique de l'ordinateur 14. Ce signal informe clairement l'opérateur des actions à accomplir, à savoir, selon le cas
- conserver la lame porte-objet 3 telle quelle, lorsqu'il s'avère que la coloration est satisfaisante ou
- soumettre la lame porte-objet 3 à une nouvelle étape de coloration avec une solution rafraîchie, ou
- détruire la lame porte-objet 3 lorsqu'il s'avère qu'elle est irrécupérable.

## Revendications

1. Installation (1) pour le contrôle de l'origine et/ou de la qualité de coupes (2) d'échantillons biologiques (7), stockées sur des lames porte-objet (3) munies de données d'identification encodées (5) et prélevées sur des blocs de paraffine (4) également munis de données d'identification encodées (8), ladite installation (1) comportant
- au moins un dispositif de lecture de données d'identification encodées, **caractérisée en ce qu'**elle comporte en outre :
- au moins un dispositif d'acquisition de données biométriques propres à un échantillon biologique (7) inclus dans un bloc de paraffine (4) ou propres à un échantillon biologique (7) stocké sur une lame porte-objet (3) sous forme d'une coupe (2) prélevée à partir d'un bloc de paraffine (4),
- des moyens de transmission (13) des données d'identification relevées et des données biométriques acquises vers des moyens de traitement informatiques intégrant une application logicielle exécutée pour
* enregistrer de manière associée dans des moyens mémoire les données d'identification relevées sur un bloc de paraffine (4) et les données biométriques propres à l'échantillon biologique (7) inclus dans ledit bloc de paraffine (4) et préalablement acquises,
* enregistrer les données d'identification relevées sur une lame porte-objet (3) et les comparer avec celles enregistrées pour ledit bloc de paraffine (4), et, le cas échéant
* enregistrer les données biométriques, propres à l'échantillon biologique (7) stocké sous forme de coupe (2) sur ladite lame porte-objet (3), préalablement acquises, et les comparer avec les données biométriques enregistrées de manière associée avec lesdites données d'identification dudit bloc de paraffine (4), et
* activer des moyens de genèse d'un signal représentatif du résultat de la ou des comparaisons.

2. Installation (1) selon la revendication 1, **caractérisée en ce qu'**elle comporte :
- Un appareil (10) présentant un logement (11) conformé pour recevoir au moins un bloc de paraffine (4) ou au moins une lame porte-objet (3) et dans lequel sont installés de manière appropriée au moins un dispositif (9) de lecture de données encodées destiné à relever les données d'identification d'un bloc de paraffine (4), au moins un second dispositif (12) de lecture de données encodées destiné à relever les données d'identification d'une lame porte-objet (3), ainsi qu'au moins un dispositif d'acquisition de données biométriques destiné à acquérir les données biométriques propres à un échantillon biologique (7) inclus dans un bloc de paraffine (4) et/ou les données biométriques propres à un échantillon biologique (7) stocké sous forme de coupe (2) sur une lame porte-objet (3) .

3. Installation selon la revendication 2, **caractérisé en ce que** ledit appareil (10) comporte un dispositif d'acquisition de données biométriques destiné à acquérir les données biométriques propres à un échantillon biologique (7) inclus dans un bloc de paraffine (4) et un dispositif d'acquisition de données biométriques destiné à acquérir les données biométriques propres à un échantillon biologique (7) stocké sous forme de coupe (2) sur une lame porte-objet (3) .

4. Installation (1) selon l'une quelconque des revendications 2 ou 3, **caractérisée en ce qu'**elle comporte en outre un ordinateur (14) pourvu des moyens de traitement informatiques et d'au moins une interface utilisateur, relié audit appareil (10) par une liaison (13) filaire ou non filaire.

5. Installation (1) selon l'une quelconque des revendications 2 à 4, **caractérisée en ce que** ledit appareil (10) intègre les moyens de traitement informatiques et au moins une interface utilisateur.

6. Installation (1) selon l'une quelconque des revendications précédentes, **caractérisée en ce que** le/les dispositif(s) d'acquisition(s) de données biométriques est/sont défini(s) par un dispositif d'acquisition de données relatives à une forme bidimensionnelle ou tridimensionnelle propre à un échantillon biologique (7) inclus dans un bloc de paraffine (4) ou propre à un échantillon biologique (7) stocké sur une lame porte-objet (3) sous forme d'une coupe (2) prélevée à partir d'un bloc de paraffine (4).

7. Installation (1) selon l'une quelconque des revendications précédentes, **caractérisé en ce qu'**elle comporte au moins un lecteur de codes-barres (9, 12).

8. Installation (1) selon l'une quelconque des revendications 4 à 7, **caractérisée en ce que** l'interface utilisateur consiste en un écran (15).

9. Procédé de contrôle de l'origine et/ou de la qualité de coupes (2) d'échantillons biologiques (7), prélevées sur des blocs de paraffine (4) munis de données d'identification encodées (5), lesdites coupes (2) étant stockées sur des lames porte-objet (3) également munies de données d'identification encodées (8), au moyen d'une installation (1) selon l'une quelconque des revendications précédentes, **caractérisé par** la réalisation, dans l'ordre, des étapes suivantes :
- relever les données d'identification d'au moins un bloc de paraffine (4) au moyen d'un dispositif de lecture (9) de données encodées,
- acquérir les données biométriques propres à l'échantillon biologique (7) inclus dans ce même bloc de paraffine (4) au moyen d'un dispositif d'acquisition de données biométriques,
- enregistrer de manière associée les données d'identification (5) du bloc de paraffine (4) et les données biométriques propres à l'échantillon biologique (7) inclus dans ce même bloc de paraffine (4) dans des moyens-mémoire,
- relever les données d'identification d'au moins une lame porte-objet (3) au moyen d'un dispositif de lecture de données encodées (12),
- acquérir les données biométriques propres à l'échantillon biologique (7) stocké sous forme de coupe (2) dans ladite lame porte-objet (3) au moyen d'un dispositif d'acquisition de données biométriques,
- comparer les données d'identification de ladite lame porte-objet (3) et les données d'identification dudit bloc de paraffine (4) puis, comparer les données biométriques propres à l'échantillon biologique (7) stocké sous forme de coupe (2) dans ladite lame porte-objet (3) et les données biométriques propres à l'échantillon biologique (7) inclus dans ledit bloc de paraffine (4),
- réceptionner un signal représentatif du résultat des comparaisons effectuées, et, selon le cas
- Confirmer l'origine et/ou la qualité de la lame porte-objet, ou
- Infirmer l'origine et/la qualité de la lame porte-objet.

10. Procédé de contrôle de la qualité de la coloration d'une lame-objet (3) au moyen d'une installation (1) selon l'une quelconque des revendications 1 à 8, **caractérisé par** la réalisation des étapes suivantes :
- relever les données d'identification d'au moins une lame porte-objet, dite « lame porte-objet de référence », au moyen d'un dispositif de lecture de données encodées,
- acquérir les données biométriques propres à l'échantillon biologique (7) stocké sous forme de coupe (2) convenablement colorée dans la lame porte-objet « de référence », au moyen d'un dispositif d'acquisition de données biométriques,
- enregistrer de manière associée les données d'identification de la lame porte-objet de référence et les données biométriques propres à l'échantillon biologique (7) stocké sous forme de coupe (2) convenablement colorée dans la lame porte-objet « de référence », dans des moyens-mémoire,
- relever les données d'identification d'une lame porte-objet (3) au moyen d'un dispositif de lecture de données encodées,
- acquérir les données biométriques propres à l'échantillon biologique (7) stocké sous forme de coupe (2) colorée dans ladite lame porte-objet (3) au moyen d'un dispositif d'acquisition de données biométriques,
- réceptionner un signal représentatif du résultat d'une comparaison entre les données biométriques propres à l'échantillon biologique (7) stocké sous forme de coupe colorée (2) dans ladite lame porte-objet (3) et les données biométriques propres à l'échantillon biologique (7) stocké sous forme de coupe (2) convenablement colorée dans ladite lame porte-objet « de référence », effectuée(s) par des moyens de traitement informatique, et, selon le cas
- Confirmer la qualité de la coloration de l'échantillon biologique (7) stocké sous forme de coupe colorée (2) dans la lame porte-objet (3) et conserver la lame porte-objet (3) telle quelle, ou
- Infirmer la qualité de la coloration de l'échantillon biologique (7) stocké sous forme de coupe colorée (2) dans la lame porte-objet (3) et soumettre la lame porte-objet (3) à une nouvelle étape de coloration, ou détruire la lame porte-objet (3).

11. Utilisation de l'installation (1) selon l'une quelconque des revendications 1 à 8 pour contrôler l'origine et/ou la qualité de coupes (2) d'échantillons biologiques, prélevées sur des blocs de paraffine (4) et stockées sur des lames porte-objet (3) ou pour contrôler la coloration de coupes (2) d'échantillons biologiques (7) stockées sur des lames porte-objet (3).
